# EUROPÄISCHE PATENTSCHRIFT

(11) **EP 1 242 834 B1**
(45) Veröffentlichungstag und Bekanntmachung des Hinweises auf die Patenterteilung: **21.01.2004**
(21) Anmeldenummer: 00916862.6
(22) Anmeldetag: 24.02.2000
(51) Int. Cl.: G01V 9/00, G01V 3/08

(54) **VERFAHREN ZUR DETEKTION VON SPURENSTOFFEN UND/ODER UMGEBUNGSEIGENSCHAFTEN**
METHOD FOR DETECTING TRACE SUBSTANCES AND/OR ENVIRONMENTAL PROPERTIES
PROCEDE DE DETECTION DE SUBSTANCES A L'ETAT DE TRACE ET/OU DE PROPRIETES DE L'ENVIRONNEMENT

(30) Priorität: 24.03.1999 DE 19913220
(43) Veröffentlichungstag der Anmeldung: 25.09.2002
(73) Patentinhaber: GSF-Forschungszentrum für Umwelt und Gesundheit GmbH, 85764 Oberschleissheim (DE)
(72) Erfinder: ZIMMERMANN, Ralf, D-80339 München (DE); KETTRUP, Antonius, D-59821 Arnsberg (DE)
(86) Internationale Anmeldenummer: PCT/EP2000/001501
(87) Internationale Veröffentlichungsnummer: WO 2000/057212

(56) Entgegenhaltungen:
- EP-A- 0 477 167
- EP-A- 0 613 027
- WO-A-94/29752
- DE-A- 3 716 846
- DE-C- 4 313 773
- US-A- 5 514 963
- BROSSIA C E ET AL: "Low-cost in-soil organic contaminant sensor" CHEMICAL, BIOCHEMICAL AND ENVIRONMENTAL FIBER SENSORS II, SAN JOSE, CA, USA, 19-21 SEPT. 1990, Bd. 1368, Seiten 115-120, XP000916212 Proceedings of the SPIE - The International Society for Optical Engineering, 1991, USA ISSN: 0277-786X

## Beschreibung

Die Erfindung betrifft ein Verfahren zur Detektion von Spurenstoffen und/oder Umgebungseigenschaften.

Die Zusammensetzung der Fraktion leicht- bis mittelflüchtiger Verbindungen als Funktion des Ortes kann Auskunft über z. B. unter dem Erdreich verbogenen Gegenstände geben. Nach diesen "Suchprinzip" können beispielsweise speziell trainierte Hunde verborgene Gegenstände wie z.B. Minen oder Drogen suchen. Werden beispielsweise Altlasten im Boden gesucht so nimmt man nach einem bestimmten Suchmuster Bodenproben, die dann in einem chemischen Labor auf z.B. Sprengstoffrückstände oder polyzyklische Aromaten (PAK) untersucht werden.

Bisher existiert kein System zur schnellen Beprobung und automatischen Analyse von großflächigen Gebieten. Wird in einem bestimmten Gebiet z. B. eine Altlast vermutet so werden Stichproben (z. B. Bodenproben) entnommen. Diese Proben werden in einem chemischen Labor im allgemeinen mit Lösungsmittel extrahiert. Der Extrakt wird dann vorgereinigt ("clean-up"-Schritt) und aufkonzentriert. Anschließend erfolgt die Analyse chemischer Spezies mit einem instrumentellen analytischen Verfahren wie z. B. mit Gaschromatographie-Massenspektrometrie (GC-MS). Zur Bestimmung der Konzentration von Verbindungen in der Luft werden meist aktive Sammelverfahren verwendet, die einen bestimmten Volumenstrom zu analysierender Luft über eine Sammeleinheit (z. B. mit Altivkohle gefüllte Adsorbtionsöhrchen oder Impinger gefüllt mit Lösungsmittel zur Absorption). Passive Sammeleinheiten werden beispielsweise zur Langzeitbeprobung von Kamingas (Emissionsüberwachung) eingesetzt.

Eine schnelle Methode zur Analyse von Luftproben stellt die Thermodesorption **[**1**]** des Sammelmediums dar. Dabei werden die vom Sammelmedium desorbierten, gasförmigen Verbindungen direkt in eine Spurenanalyseinstrument übertragen. Für leichtflüchtige Verbindungen haben sich beispielsweise verschiedene Aktivkohlemodifikationen (z. B. Cabotrap) oder Kunstharze (z. B. TENAX) **[**2**]** bewährt. Für die Thermodesorptionsanalyse schwerer flüchtiger Verbindungen sind z. B. Sammeleinheiten aus Silikon-Gummi geeignet **[**3**]**, **[**4**]**.

Direkte Methoden zur Bestimmung der Konzentrationsverteilung chemischer Verbindungen über einem Gelände existierten zwar prinzipiell (z. B. LIDAR), sind aber im allgemeinen zu unempfindlich und unspezifisch um zur indirekten Detektion von verborgenen Gegenständen eingesetzt werden zu können.

DE-A-3 716 846 offenbart ein Verfahren zur Überwachung von Gebieten, z.B. Mülldeponien, bei dem Eigenpotentialmessungen an der Oberfläche des Gebietes durch mehrere Basissonden und eine Vielzahl von Potentialsonden vorgenommen werden. Fig. 1 dieses Dokumentes zeigt einige Reihen (6-9) von Potentialsonden, die in dem zu überwachenden Gebiet aufgestellt sind, wobei die Sonden in Bodenlöcher eingesetzt werden.

Aufgabe der Erfindung ist es, ein Verfahren der e. g. Art für eine schnelle, positionsaufgelöste Analyse anzugeben.

Gelöst wird diese Aufgabe durch die Merkmale des Patentanspruchs 1. Die Unteransprüche beschreiben vorteilhafte Ausgestaltungen des Verfahrens.

Die Erfindung wird im folgenden anhand von Ausführungsbeispielen mit Hilfe der Figuren näher erläutert.

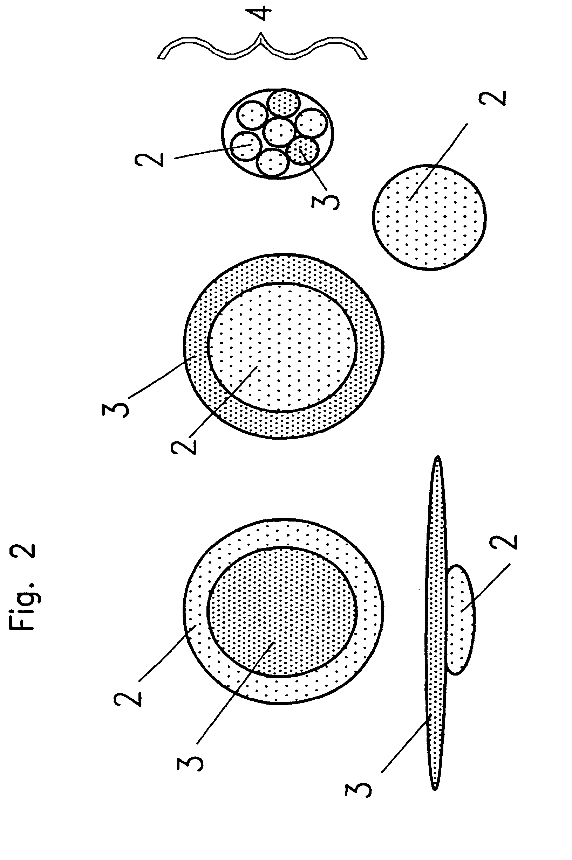

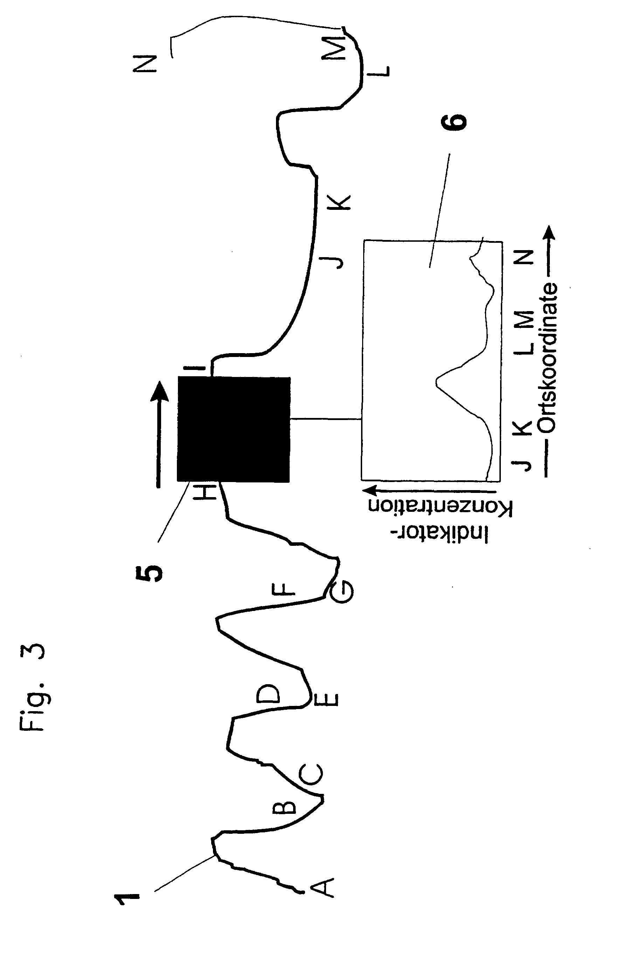

Dabei zeigt die Figur 1 eine Auslegung des Sammelmediums und die Figur 2 verschiedene mögliche Ausgestaltungen des Sammelmediums 1. Die Figur 3 zeigt die Zuordnung der Stoffkonzentration zu Ortskoordinaten. Die Figur 4 a und b zeigt zwei mögliche Verlegunsschemata für den Zweck der Suche nach Sprengstoffen und Mienen. Die Figur 5 zeigt eine Verlegefahrzeug für das Sammelmedium 1.

Auf der zu untersuchenden Fläche wird ein schnurförmiges Sammelmedium 1 nach einem Ortsraster ausgelegt. Das schnurförmiges Sammelmedium 1 besteht ganz oder teilweise aus einer Substanz 2, die chemische Verbindungen aus der Umwelt anlagern, aufnehmen oder anreichern kann oder auf Umwelteinflüsse reagieren kann. Nach einer bestimmtem Einwirkungszeit haben sich z. B. flüchtige organische Verbindungen auf den Sammelmedium entsprechend ihrer lokalen Umgebungskonzentration angelagert oder angereichert. Nach dem Einholen der Schnur 1 kann die Konzentration angereicherter Verbindungen in einem automatischen Analyseinstrument 5 als Funktion der Position auf der Schnur analysiert werden. Bringt man die chemische Information 6 entlang des Sammelmediums mit dem Ortsraster in Zusammenhang so erhält man eine "Landkarte" der chemischen Information. Je nach Anwendungsgebiet kann die chemische Information z. B. auf die Position von Altlasten oder Bodenschätzen hinweisen. Für einen wirtschaftlichen Einsatz ist dabei wichtig, daß ein besonders kostengünstiges Sammelmedium, welches als Massenware erhältlich ist und günstige physikalische Eigenschaften hat (z. B. hohe Reißfestigkeit) eingesetzt werden kann. Beispielsweise kommen dafür beschichtete oder unbeschichtete Polymerschnüre z. B. aus Nylon in Frage.

### VERFAHREN ZUR ORTSAUFGELÖSTEN DETEKTION VON GASFÖRMIGEN ODER GELÖSTEN SPURENSTOFFEN: KONZEPT

Durch Auslegen eines flexiblen, band- oder schnurförmigen Sammelmediums nach einem Ortsraster auf einer zu untersuchenden Fläche werden während einer Einwirkzeit ortsaufgelöste Informationen über die untersuchte Umgebung gesammelt. Das band- oder schnurförmige Sammelmedium ist zur Adsorption und/oder zur Absorption der nachzuweisenden Spurenstoffe oder zur chemischen oder biologischen Umsetzung der nachzuweisenden Spurenstoffe oder zur chemischen, physikalischen oder biologischen Indikation physikalischer, chemischer oder biologischer Eigenschaften geeignet und dient somit als Passivsammler/indikator für flüchtige organische oder anorganische Verbindungen oder andere Umweltbedingungen.

Prinzipiell gibt es zwei Möglichkeiten:
1. Bei der Auslegung des band- oder schnurförmigen Sammelmediums als "ortsauflösender chemischer Sammler" werden Verbindungen aus der Umwelt an das Sammelmedium gebunden 2 bzw. auf oder in diesem angereichert. Dieses erfolgt durch Ad- oder Absorption (Physi- oder Chemisorption). Diese auf dem Sammelmedium befindlichen Analyten werden nach dem Einholen in einem analytischen Detektionsschritt positionsaufgelöst bestimmt.
2. Bei der Auslegung des band- oder schnurförmigen Sammelmediums als "ortsauflösender Indikator" werden Eigenschaften des band- oder schnurförmigen Sammelmediums 1 betrachtet, die sich als Folge der Umwelteinflüsse ändern können. Beispielsweise können chemisch, biologisch oder physikalisch arbeitende Indikatoren in die Sammelschnur eingearbeitet sein.

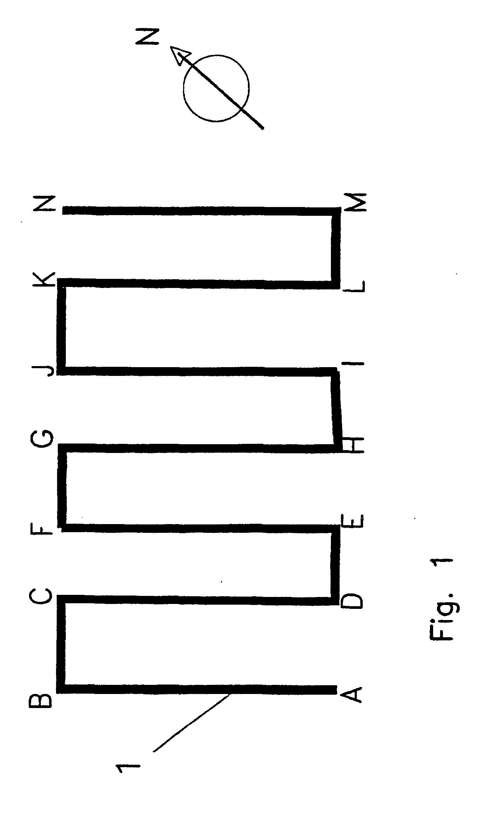

Analyte aus der Luft (oder aus dem Wasser) über der Fläche werden angereichert. Das definierte Ortsraster erlaubt es dann, bei der späteren positonsaufgelösten Analyse die mittlere Konzentration als Funktion der Auslegeposition zu ermitteln. Figur 1 zeigt schematisch, wie des Auslegen des Sammelmediums 1 auf einer Fläche (Feld, Fabrikgelände etc.) zur Detektion z. B. von verborgenen Schadstoffen (z. B. von Altlasten und Produktionsrückständen), militärischem Material (Minen) oder Lagerstätten fossiler Brennstoffe erfolgen kann. Nach einer Einwirkzeit wird das flexible Sammelmedium 1 eingeholt. Die angereicherten Verbindungen können als Funktion der Position auf dem Sammelmedium 1 analysiert werden. Die Analyse kann mit eine Reihe verschiedener Verfahren durchgeführt werden. Besonders geeignet sind in diesem Zusammenhang on-line Meßverfahren die, z. B. über einen dazwischen geschalteten Thermodesorptionsschritt, eine direkte Analyse von Verbindungen als Funktion des Ortes auf dem Sammelmedium 1 erlauben. Eine Reihe von feldtauglichen on-line Meßverfahren sind beschreiben worden [5], [6]. Anhand des Ortsrasters kann dann die Konzentration der chemischen Verbindungen im Luft/Bodenraum dem Ort auf der untersuchten Fläche zugeordnet werden.

### DETAILBESCHREIBUNG DER KOMPONENTEN

### a) Das Sammelmedium

Die sammelaktive Substanz 2 des Sammelmediums 1 kann entsprechend der gewünschten Anwendung modifiziert werden und ist somit für eine Vielzahl von Analyte einsetzbar.

Die Anreicherung erfolgt ad-, absorbtiv oder reaktiv (Chemisorption). In der *Solid Phase Micro Extraction* (SPME) Technik für die Gaschromatographie ist gezeigt worden welche hohe Anreicherungsfaktoren mit einer passiven Sammelmethode erreicht werden. Auf einer 10 mm langen Quarzfaser mit einer 10 - 100 µm dicken Beschichtung aus z. B. Phenylsiloxan lassen sich so in kurzer Zeit Verbindungen anreichern die dann im GC nachgewiesen werden. Nachweisgrenzen für chemische Verbindungen liegen dabei häufig im ppt-Bereich **[**7**]** oder besser.

Als sammelaktive Substanzen 2 kommen z. B. Polymere und Beschichtungen in Frage die in der Gaschromatographie als stationäre Phasen eingesetzt werden. Beispiele sind Polyacrylat, Polysiloxan, Polydimethylsiloxan, Phenylsiloxan, Methylsioxan oder Carbowax. Weiterhin sind auch Polymere wie Teflon, Nylon etc. geeignet.

Unpolare Verbindungen reichern sich durch Eindiffussion und Lösung in unpolaren Medien wie z. B. Silikon an. Beispielsweise kann eine mit Silikon beschichtete Perlonschnur eingesetzt werden um unpolare, mittelflüchtige Analyte anzureichern. Silikon eignet sich besonders zur Detektion mittelflüchtiger Verbindungen. Wenn z. B. leichterflüchtige Verbindungen miterfaßt werden sollen kann ein weiters Medium wie z. B. Aktivkohlestaub *(Carbotrap* etc.) dem Silikon beigemischt werden. Alternativ kann eine silikonbeschichtete Carbonfaser eingesetzt werden.

Polare organische Verbindungen benötigen andere Sammelmedien (die selber polarer sind). Beispielsweise können Siloxan Phasen durch Einführen von Cyanid-Gruppen (-CN) oder die Beimischung entsprechender sammelaktiver Stoffe z.B. in eine Silikon Matrix.

Die Anreicherung ionischer Verbindungen oder Metallionen aus der wäßrigen Phase (Probenahme aus dem Wasser oder aus dem Boden) kann nach dem Prinzip des Ionenaustausches erfolgen. Mit Ionenaustauscherharzen beschichtete Schnüre können somit zur ortsaufgelösten Detektion von Ionen verwendet werden.

Je nach Art der zu anzureichernden und analysierenden chemischen Substanzen können auch spezielle Sammelmedien entwickelt werden, die chemische oder biologische Reagenzien enthalten. Zur Anreicherung ionischer, polarisierbarer oder polarer Verbindungen können beispielsweise komplexierende Verbindungen (z. B. Chelatbildner wie EDTA für Metallionen) in einer Matrix eingesetzt werden.

Im Sammelmedium 1 eingebrachte Reagenzien, die mit bestimmten Analyten unter Änderung absorptionsphotometrischen Eigenschaften (Farbreagenzien) oder der Fluoreszenzeigenschaften (Fluoreszenz - Marker) reagieren können eingesetzt werden. Das Sammelmedium 1 kann dann sehr einfach und schnell über spektroskopische Detektion (IR- oder UV/VIS Absorption/Reflektion, Luminiszenzdetektion etc.) "abgescannt" werden. Nach einer solchen "zerstörungsfreien" Detektion kann die Sammelschnur 1 noch weiteren Analysenverfahren zugeführt werden.

Bei den biologischen Reagenzien kann es sich beispielsweise um antikörperbasierte Verfahren handeln (z. B. wie beim Immuno: Essay Verfahren). Werden lebende oder lebensfähige Mikroorganismen oder Sporen (Bakterien, Pilze, Algen, andere Einzeller etc.) in des Medium 1 eingelagert (oder angelagert etc.) so kann ein ortsaufgelöster "ökotoxikologischer" Test durchgeführt werden, indem die Stoffwechselprodukte, das Wachstum oder das Absterben oder andere Eigenschaften der Zellen untersucht werden. Je nach Enzymausstattung der Mikroorganismen kann eine spezifische Detektion bestimmter essentieller oder toxischer Stoffe erreicht werden. (Manche Bakterien veratmen z. B. H₂S oder Metallspezies oder benötigen einen bestimmten pH Wert oder eine bestimmte O₂-Konzentration zum Wachsen. Außerdem können Stämme eingesetzt werden die, z. B. gegen ein bestimmtes Xenobiotika besonders empfindlich sind.

Die positionsaufgelöst angereicherten Stoffe können auch über eine externe immunologische oder biologische Methode detektiert werden. Das Sammelmedium 1 kann mit einem anderen Medium in Kontakt gebracht werden, so daß zwischen den Medien unter Erhalt der Positionsauflösung Reagenzien und/oder Analyte ausgetauscht werden können (z. B. Übertragung auf Immuno-Essay-Trägerplatten etc.). Alternativ kann die Sammelschnur 1 abschnittsweise auch naß-chemisch extrahiert werden und dann einer chemischen, biologischen, physikalischen oder immunologischen Analyse zugeführt werden.

Weiterhin können neuere Techniken, die auf einer künstlichen Herstellung von Rezeptoren beruhen (z. B. Molecular Imprinting), eingesetzt werden **[8].**

Wenn mehrere spezifisch präparierte schnur- oder faserförmige Sammelmedien 1 eingesetzt werden, können diese zu einem Bündel zusammengestellt werden. Wenn die Fasern aus verschiedenen sammelaktiven Substanzen 2 bestehen bzw. unterschiedlich beschichtet sind, dann können viele verschiedene Analyte (auch chemisch verschiede: z. B. polare und unpolare Verbindungen) gleichzeitig angereichert werden. Weiterhin können mit solchen "Multifaser-Sammelmedien größere Konzentrationsbereiche der Analytsubstanzen wiedergegeben werden, da quantitative Ergebnisse für jedes Sammelmedium nur in einem gewissen Konzentrationsbereich erhalten werden können.

Wenn das sammelaktive Material auf der Unterseite eines Bandes aufgebracht wird oder die verlegte Schnur mit einem Band z. B. aus Kunststoff abgedeckt wird so können die aus dem Boden permeierende chemische Information nicht durch Wind etc. verschleiert werden.. Die Einwirkzeit ist je nach dem zu erwartenden Konzentrationsbereich zu wählen und wird meistens im Bereich von Stunden bis Tagen liegen. Nach der Einwirkungszeit wird das Sammelmedium eingeholt.

Für eine Feldanwendung des Verfahrens kann es vorteilhaft sein, wenn das Sammelmedium 1 seine sammelaktive Wirkung erst beim Auslegen entfalten kann um Kontaminationen zu vermeiden. Ähnlich könnte das Sammelmedium 1 nach dem Einholen und vor einer Analyse (z. B. wenn eine Lagerung nötig ist) deaktiviert werden. Eine Möglichkeit dies zu erreichen ist die Verwendung eines elastischen Sammelmedium 1 das aus einem sammelaktiven Kern und einer für die zu analysierenden Verbindungen nicht oder nur schlecht durchlässigen Beschichtung besteht, die bei einer Dehnung des elastischen Sammelmediums 1, z. B. während der Probennahme, durchlässiger für bestimmte Analyte wird und in zusammengezogener Form, z. B. während des Transports zum Analysegerät oder der Lagerung, weniger durchlässig für die Analyte ist.

Es ist von Vorteil das Sammelmedium nach der Aufnahme so zu behandeln, daß die Information möglichst unverfälscht vorhanden bleibt. Dies kann beispielsweise durch Kühlung und oder Lagerung des Sammelmediums 1 in einer inerten Atmosphäre erreicht werden. Weiterhin ist eine Fixierdung der Information beispielsweise durch chemische Reagenzien möglich. Eine weiter Möglichkeit bieten der Überzug eine undurchlässigen Schicht. Eine Beschichtungsvorrichtung kann in eine Gerät integriert sein, mit dem das Sammelmedium 1 nach dem Auslegen wieder aufgenommen wird.

### b) Der Analysator

Die auf dem eingeholten Sammelmedium 1 positionsaufgelöst vorliegende Information muß mittels geeigneter Analyseverfahren nachgewiesen werden. Um das Verfahren zum "Abscannen" größerer Gebiete einsetzen zu können ist neben einem billigem Sammelmedium 1 erforderlich, daß die Analyse schnell und möglichst automatisch verläuft. Idealerweise sollte das Analysegerät 5 das Sammelmedium 1 automatisch einziehen und positionsaufgelöst analysieren.

Es sind prinzipiell zwei Analyseprinzipien möglich.
1. Die im Sammelmedium 1 eingelagerten Verbindungen oder die Veränderungen im Sammelmedium 1 werden direkt im Sammelmedium 1 nachgewiesen. Beispielsweise kann die Sammelschnur 1 abschnittsweise mit Laserlicht bestrahlt werden. Im Sammelmedium 1 angereicherte Aromaten werden dann durch das emittierte Fluoreszenzlicht nachgewiesen werden.
2. Die (chemischen) Information wird vor der Analyse auf eine anderes Medium übertragen. Beispielsweise kann die Sammelschnur 1 durch eine Thrmodesorptionseinheit gezogen werden. Dabei werden die angereicherten chemischen Verbindungen in die Gasphase überführt und von einer on-line analytischen Methode wie z. B. REMPI-TOFMS kontinuierlich erfaßt.

Wenn nicht destruktive Nachweis- oder Analyseverfahren angewandt werden können auch verschiedener Detektionsverfahren nacheinander ausgeführt werden.

Vor oder nach einer Themodesorption kann der Nachweis der Analyte z. B. durch folgende Verfahren erfolgen:
- UV-VIS (Absorptionsspektroskopie)
- Fluoreszenzspektroskopie
- IR (Infrarotabsorptionsspektrometrie)
- Ramanspektroskopie
- NMR (Kernresonanzspektrometrie)
- Flammenionisationsdetetkor (FID)
- Electron Capture Detector (ECD)
- Thermionic Detetcor (TID)
- Röntgenfluoreszenzspektroskopie
- Ionenmobilitätsspektrometrie [9]
- elektronischen Nasen [10] oder Sensoren [11]
- AAS (Atomabsorptinsspektrometrie)
- laserspektroskopische Verfahren
- massenspektrometrische Verfahren.

Laserspektroskopische Verfahren wie z. B. laserinduzierte Fluoreszenz (LIF), laserinduzierte Plasmaspektrometrie (Laser Induced Plasma Spectroscopy (LIPS), zum Elementnachweis) oder photoakustische Spektroskopie erlauben oft besonderes hohe Selektivitäten und Empfindlichkeiten detektiert werden. Im folgenden werden exemplarische einige möglich Nachweisverfahren genauer vorgestellt. Das Sammelmedium kann zur Analyse durch eine spezielle Thermodesorptionseinheit gezogen wird, wobei die ortsaufgelöste, chemische Information auf dem schnurförmigen Sammelmedium in eine entsprechende zeitliche aufgelöste Information in einem, die Themodesorptionseinheit durchströmenden Trägergasstrom, überführt wird (Thermodesorption (TD) der Analyte). Diese, zeitlich variierende chemische Information im Gasstrom wird von einem Analysengerät oder Detektor zeitaufgelöst erfaßt. Eine neuere, sehr empfindliche und selektive Methode zum Spurennachweis z. B. von aromatischer Verbindungen aus dem TD Gasstrom stellt die resonanzverstärkte Mehrphotonenionisation (REMPI) mit anschließender Flugzeitmassenanalyse (TOFMS) dar. Der Nachweis erfolgt z. B. über eine automatischen Thermodesorptionseinheit (TD-Einheit), die on-line an ein REMPI-TOFMS Spektrometer angeschlossen ist. Die automatische TD-Einheit zieht des Sammelmedium selbsttätig ein. Das Sammelmedium wird z. B. durch ein 150 - 300° C heißes Röhrchen aus Glas gezogen und somit lokal erwärmt. Gleichzeitig fließt ein schwacher Probenaufnahme-Gasstrom (z. B. 0,1 - 10 ml pro Minute) durch das Glasröhrchen über das lokal erhitzte Sammelmedium und nimmt die thermisch desorbierten Verbindungen auf. Über einen Direkteinlaß wird dann der Probenaufnahmegasstrom direkt in das REMPI-TOFMS Spektrometer eingeleitet. Zu den Einlaßmöglichkeiten für REMPI-TOFMS siehe ZITATE **[**12**]**. Da die Nachweisempfindlichkeit für viele aromatische Systeme im pptv-Konzentrationsbereich liegt, ist ein sehr empfindlicher Nachweis möglich. Weiterhin ist eine Verwendung von on-line Derivatisierungsschritten für REMPI-TOFMS möglich um bestimmte Verbindungen nachzuweisen **[**13**]**. Sollte die Nachweisempfindlichkeit für bestimmte Indikator- oder Zielverbindungen nicht ausreichen kann eine weitere Anreicherung, allerdings unter Verringerung der örtlichen Auflösung, vorgenommen werden. Dabei werden die angereicherten Verbindungen einer bestimmten Länge des Sammelmediums 1 integrativ analysiert. Ein mögliche Realisierung kann eine Spule beinhalten, auf der eine gewisse Menge des Sammelmediums aufgerollt wird. Danach erfolgt die simultane thermische Desorption (und anschließende Analyse der Analyte) des gesamtem aufgerollten Sammelmediums. Anschließend wird die Spule abgewickelt und die nächste Länge des noch nicht analysierten Sammelmediums aufgerollt, thermodesorbiert usw. Alternativ kann bei kontinuierlicher Thermodesorption eine periodisch thermodesobierbare Kältefalle in den Probenaufnahme-Gasstrom eingefügt werden.

Die Desorption der chemischen Verbindungen, die auf dem schnurförmigen Sammelmedium angereichert sind kann bei der Analyse in der Probennahmeeinrichung eines Analysegerätes durch Bestrahlung mit Laserpulsen erfolgen. Diese Laserdesorption der Analyte kann direkt im Vakuumbereich eines Massenspektrometers erfolgen. Wird das Sammelmedium dabei automatisch durch das Analysegerät gezogen so entspricht daß Verfahren einer Art on-line MALDI **[**14**]** - oder Laser-Microprobe Analysis (LAMMA) **[**15**]** Technik. Eine geeignete MALDI Matrix kann im Sammelmedium enthalten sein. Alternativ kann der Laser nur zur Desorption eingesetzt werden und die Ionisation über eine andere Methode erfolgen (REMPI, EI, FAB etc.) **[**16**]**.

### BESCHREIBUNG MÖGLICHER ANWENDUNGSBEISPIELE

Das Verfahren hat eine Vielzahl möglicher Anwendung. Die ortsaufgelösten Detektion von organischen oder anorganischen Verbindungen kann beispielsweise für folgende Anwendungen eingesetzt werden:
- Lokalisation von Altlasten wie z. B. Produktionsrückständen auf ehemaligen Fabrikgeländen (BTX, PAK, Schwermetalle, PCB etc.)
- Detektion militärischen Altlasten, wie z. B. von Produktionsrückständen, Explosivstoffen, Mienen (z. B. TNT, RDX etc.)
- Forensische Anwendungen z. B. zur gezielten Suche nach Drogen oder Leichen
- Detektion geologischer Lagerstätten von z.B. Erdgas, Erdöl (über Kohlenwasserstoffe) oder Erzen (z. B. über Metallionen)
- Ermittlung von Konzentrationsprofilen chemischer Verbindungen innerhalb von Industrieanlagen oder entlang von Rohrleitungen z. B. zur Detektion von Leckagen entlang von Pipelines.
- Ermittlung anthropogener oder bio-/geogener Emissions- oder Imissionskonzentrationen chemischer Substanzen oder anderer chemischer, physikalischer oder biologischer Eigenschaften in Wasser, Land (Boden) oder Luft (z. B. Langzeitüberwachung von Vulkan-/Erdbebenaktivität durch Registrierung von vulkanischen Gasen wie H2S oder CO2, Ermittlung der biogenen Flächenemission von CH4, N2O etc.).

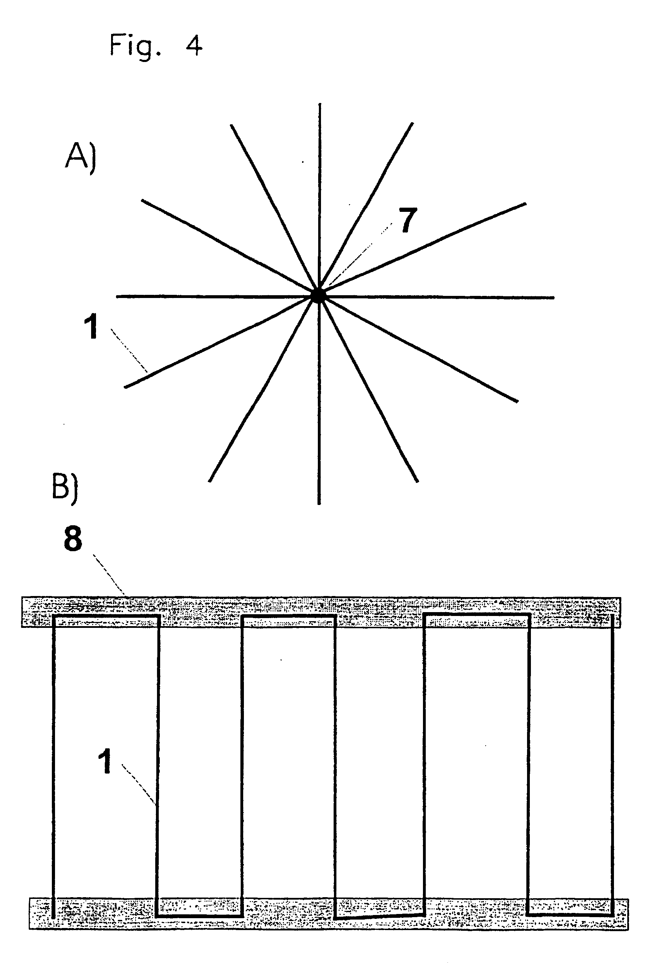

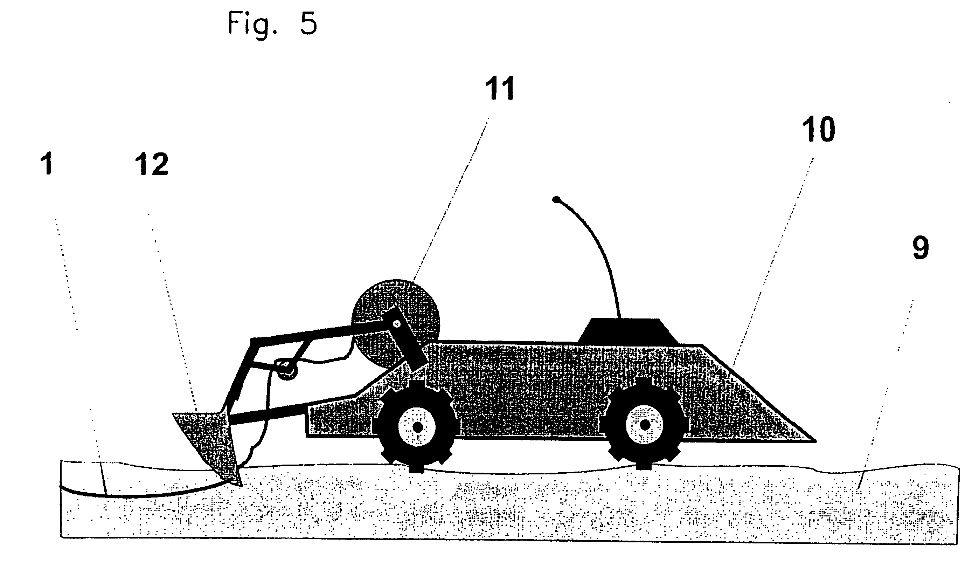

Im folgenden sind einige Anwendungen ausführlicher diskutiert.
**a) Detektion von chemischen Altlasten und Produktionsrückständen** Durch Auslegen eines flexiblen, band- oder schnurförmigen Sammelmediums als passiver Sammler für flüchtige organische oder anorganische Verbindungen nach einem Ortsraster über einer zu untersuchenden Fläche werden Analyte aus der Luft (oder aus dem Wasser) über dieser Fläche angereichert. Das definierte Ortsraster erlaubt dabei, bei der späteren positonsaufgelösten Analyse die Konzentration als Funktion der Auslegeposition zu ermitteln. Die Figur 1 zeigt schematisch wie des Auslegen des Sammelmediums 1 beispielsweise auf einem ehemaligen Fabrikgelände zur Detektion einer verborgenen, mineralölbasierten Altlast erfolgen könnte. Im Falle von mineralölbasierten Altlasten eignen sich monozyklische Aromaten wie z. B. Benzol, Toluol und Xylol (BTX) oder kleinere polyzyklische aromatische Kohlenwasserstoffe wie z. B. Naphthalin, Mono- und Dimethylnaphthalin, Acenaphten, Fluoren, Biphenyl oder Anthracen als Indikatoren. Als Sammelmedium sind eine Vielzahl von Medien einsetzbar. Beispielsweise kann eine mit Silikon beschichtete Perlonschnur eingesetzt werden. Da sich Silikon primär zur Detektion mittelflüchtiger Verbindungen eignet, ist es unter Umständen vorteilhaft noch ein oder mehrere Medien zur Absorption leichtflüchtiger Verbindungen (z. B. fein gemahlenes Carbotrap oder TENAX) beizumischen. Alternativ könnte eine silikonbeschichtete Carbonfaser eingesetzt werden. Weiterhin kann ein Bündel aus mehreren sammelaktiven Fasern zusammengestellt werden. Wenn die Fasern aus verschiedenen sammelaktiven Substanzen bestehen bzw. unterschiedlich beschichtet sind, kann ein größer Konzentrationsbereich der Analytsubstanzen wiedergegeben werden, da quantitative Ergebnisse für jedes Sammelmedium nur in einem gewissen Konzentrationsbereich erhalten werden können. Wenn das sammelaktive Material auf der Unterseite eines Bandes aufgebracht wird oder die verlegte Schnur mit einem Band z.B. aus Kunststoff abgedeckt wird so kann die aus dem Boden dringende chemische Information nicht durch Wind etc. verschleiert werden. Die Einwirkzeit ist je nach dem Sammel- oder Nachweisprozeß und dem zu erwartenden Konzentrationsbereich der Analyte zu wählen und wird voraussichtlich meistens im Bereich von Stunden bis Tagen liegen. Nach der Einwirkungszeit wird das Sammelmedium eingeholt. Die Analyse der chemischen Substanzen kann über eine Vielzahl von Methoden erfolgen. Aromatische Verbindungen wie BTX oder PAK können z. B. sehr gut mit resonanzverstärkter Multiphotonenionisation-Flugzeitmassenspektrometrie (REMPI-TOFMS) nachgewiesen werden. Eine relativ universale Methode zum Nachweis von flüchtigen Verbindungen ist Massenspektrometrie mit einer CI- Ionenquelle (z.B. PTR-MS [17]). Für weitere Nachweisverfahren siehe oben.
**b) Detektion von militärischen Altlasten, Sprengstoffen, chemischen Kampfstoffen oder Minen**
   Das Verfahren kann weiterhin zur ortsaufgelösten Detektion von militärischen Altlasten, Sprengstoffen, chemischen Kampfstoffen oder Minen eingesetzt werden. Die Detektion von militärischen Altlasten wie z. B. Produktionsrückständen der TNT-Produktion oder der Herstellung chemischer Kampfstoffe ist besonders in den dicht besiedelten Gebieten Mitteleuropas wichtig (z. B. zur Zeit verstärkt aktuell in den neuen Bundesländern). Bei der Untersuchung von ehemaligen TNT-Produktionsstätten reicht unter Umständen sogar ein einfacher ECD (Electron Capture Detector) oder ein thermionischer Detektor (TID, stickstoff- und phosphorselektiv) [18] zum Auslesen der chemischen Information auf der Sammelschnur aus. Ansonsten können auch hier prinzipiell eine Vielzahl verschiedener Detektionsprinzipien angewandt werden (z.B. REMPI-TOFMS, IMS, elektronische Nase, PTR-MS). Elementspezifische Detektoren wie ein Atomemissions-Detektor (AED) oder ein TID (für Stickstoff und Phosphor) [18] können auch für die Detektion chemischer Kampfstoffe eingesetzt werden.
   Ein besonderes Problem stellt die Detektion von Minen, im speziellen Anti-Personenminen dar. Im vielen Ländern Asiens, Afrikas teilweise auch Südamerikas und sogar Europas (Bosnien) finden sich weite, von Anti-Personenminen verseuchte Landstriche. Das Minenräumen ist eine sehr gefährliche Angelegenheit da eine genauere Lokalisation der Minen mit "technischen " Hilfsmitteln oft nicht möglich ist. Anti-Personenminen neuen Typs kommen fast ohne Metallteile aus, was einen Einsatz von Metalldetektoren unmöglich macht. Mit Erfolg werden jedoch Hunde eingesetzt, die das "Bukett" der Minen "erriechen" können. Dieses Geruchsmuster setzt sich aus TNT oder RDX Spuren sowie aus deren Abbauprodukten (z. B. Di- und Mononitrotoluole, Aminotoluole etc.) zusammen. Weiterhin können charakteristische Sekundärstoffe z. B. Lösungsmittelreste, Weichmacher aus der Kunststoffhülle, Kunststoffabbauprodukte (Monomere etc.) der typischerweise 0 - 20 cm tief eingegrabenen Minen von den Hunden erfaßt werden. Der Einsatz der Hunde ist jedoch sehr aufwending und langsam. Um größere Gebiete "freizumessen" bzw. um einzelne Minen grob zu lokalisieren, könnte das hier vorgeschlagene Verfahren eingesetzt werden. Die Sammelschnüre 1 können auf vielerlei Weise ausgebracht werden. Ein Möglichkeit ist der Einsatz von Räumfahrzeugen die sichere Schneisen erstellen, zwischen denen dann im Abstand von z. B. 5 oder 10 Metern Sammelschnüre 1 positioniert werden. Für unzugänglichere Gebiete mit hoher Gefährdung durch Minen könnten Abschußvorrichtungen eingesetzt werden die, einmal zum Einsatzort gebracht, z. B. 12 Sammelschnüre 1 radial-symmetrisch abschießen. Bei einer Länge der Sammelschnüre 1 von ca. 60 m läßt sich so ein Gebiet von ca. 20000 m² mit einer Mindestauflösung von 10m untersuchen. Die Abschußvorrichtung kann über z. B. Hubschrauber abgesetzt und später wieder aufgenommen werden. Wichtig ist für alle Ausbringungsverfahren die genaue und reproduzierbare Bestimmung der örtlichen Position der Sammelschnüre 1. Dies kann z. B. durch Luftaufnahmen oder Satellitenortung erleichtert werden. Die Figuren 4 und 5 zeigt zwei mögliche Ausbringungsverfahren für die Suche von Minen. Unter Umständen muß die Sammelschnur 1 auch in einer mit Erde bedeckten Furche verlegt werden. Dies könnte mittels eines (ferngesteuerten) Verlegefahrzeugs 10 realisiert werden (siehe Figur 5).
   Da die Ausdünstungsprodukte aus Minen im extremen Spurenbereich vorliegen (ppqv oder sogar darunter) ist eine extrem selektive und empfindliche Analytik für die Sammelmedien 1 nötig. Einige der weiter oben erwähnten analytischen Techniken sind prinzipiell für die Analyse der Sammelschnüre 1 geeignet. Die REMPI-Technik oder IMS^{**Fehler! Textmarke nicht definiert**}**.** beispielsweise eignen sich zur Detektion von Nitroaromaten über das NO⁺ Ion in Massenspektrum. In Ergänzung oder alternativ zu einer instrumentellen Detektion kann die Identifikation des "Minen- Buketts" nach Art der "Sniffing-Detection" bei der GC auch von abgerichteten Hunden übernommen werden. (TD-Gasstom). Durch eine reizarme Umgebung (Boxen) und Anwendung von "Token"-Systemen kann die Aufmerksamkeit der abgerichteten Tiere u. U. auf die Aufgabe konzentriert werden. Sammelschnüre 1 können auch zur Überwachung von chemischen Produktionsanlagen eingesetzt werden. Hierbei könnte z. B. die Einhaltung des Produktionsverbots chemischer Kampfstoffe, Raketentreibstoffe oder Sprengstoffe
**c) Detektion von Lagerstätten z. B. fossiler Brennstoffe oder Erze**
   Das Verfahren kann zur Detektion von Bodenschätzen, die ausreichend dicht an der Oberfläche liegen, eingesetzt werden. Bei der Suche nach fossilen Bodenschätzen (Öl, Gas, Kohle) wird nach organischen Verbindungen gesucht (CH4, BTX, PAK, Alkane oder Schwefelwasserstoff). Für den Sammelschritt z. B. von leichtflüchtigen Verbindungen wie H2S oder Methan müssen Sammelmedien 1 mit hoher Absorptionskraft eingesetzt werden (z. B. Graphit in Siloxanmatrix auf einem Trägermaterial. Die Detektion der organischen Verbindungen erfolgt dann wie oben beschrieben. Bei der Suche nach mineralischen Bodenschätzen (Kupfer, Mangan- oder Uranerz) wird im allgemeinen nach den entsprechenden Metallionen aus der wäßrigen Phase oder aus dem (feuchten) Boden gesucht werden. Die Suche kann somit prinzipiell über oder unter Wasser durchgeführt werden. Als Sammelprinzip für ionische Verbindungen sind Ionenaustauscher besonders geeignet. Die Detektion kann z. B. mit laserspektrometrischen Methode (Laserinduzierte Plasmaspektroskopie, LIPS) oder ICP- MS erfolgen.

### Abbildungen

### Figur 1:

Das Auslegen des schnurförmigen Sammelmediums 1 (passiver Sammler) im Gelände nach einem Ortsraster erlaubt die ortsaufgelöste Detektion von Spurenstoffen.

### Figur 2:

Die Sammelschnur 1 kann aus einer Trägerschnur 3 (z. B. einer Polymerschnur oder einem Stahldraht) besteht, die mit einer oder mehreren Schichten gleicher oder verschiedener sammelaktiver Substanzen 2 wie z. B. mit Silikon TENAX oder Aktivkohle beschichtet ist. Alternativ kann die Sammelschnur 1 aus einem sammelaktiven Kern 2 bestehen, der von einem tragendem (und schützendem) Medium 3 umgeben ist. Verschiedenes sammelaktive und tragende Fasern 2 und 3 können auch zu einem Faserbündel 4 zusammengesetzt werden. Unter Umständen besteht das Sammelmedium 1 auch nur aus einem sammelaktiven Material 2 (z. B. einer Polymerschnur). Das Trägermaterial 3 kann auch als Band ausgebildet sein um die sammelaktive Substanz 2 vor Beeinflussung von z. B. Wind und Wetter zu schützen.

### Figur 3

Das Sammelmedium 1 wird z. B. mit einem automatischen on-line Analysegerät 5 ausgelesen, das die Sammelschnur selbsttätig einzieht. Dieses Analysegerät 5 kann beispielsweise eine Thermodesorption-Lasermassenspektrometrie Einheit (TD-REMPI-TOFMS) darstellen. Das Ergebnis der Messung ist eine Ortskoordinaten-Konzentrations Diagramm 6, welches eine Korrelation der Meßwerte mit dem Ortsraster (Figur 1) erlaubt.

### Figur 4

Beim Einsatz des Verfahrens zur Detektion von Minen oder Explosivstoffen ist die gefahrlose Ausbringung der Sammelschnüre 1 wichtig. Beispielsweise können aus der Luft (Hubschrauber etc.) eine Einheit 7 abgesetzt werden, welche die Sammelschnüre 1 radial auschleudert/abschießt. Nach der Einwirkzeit kann die Einheit 7 wieder aus der Luft aufgenommen werden. Auf zugänglichem Gelände können mit schwerem Räumgerät sicher Schneisen 8 generiert werden, zwischen denen die Sammelschnüre 1 gespannt werden können.

### Figur 5

Für manche Anwendungen kann es nötig sein, daß die Sammelschnur 1 in Erdfurchen oder unter der Erde 9 verlegt werden muß. Um dies zu realisieren kann die Schnur besipielsweise von einem Fahrzeug 10 verlegt werden. Über ein Art Pflugschaufel 12 kann das Sammelmedium 1 direkt von einer Voratsrolle 11 in den Boden 9 eingebracht werden. Für die Anwendung im Bereich Minensuche könnte das Fahrzeug 10 mit einer Fernsteuerung ausgerüstet sein.

### Literaturzitate

**[1]** M. Blumenstock, R. Zimmermann, K.-W. Schramm, A. Kaune, U. Nikolai, D. Lenoir, A. Kettrup, Organohalogen Compounds Vol. 36 (ISBN 91-89192-05-2), Edited by the Swedisch Environmental Protection Agency (1998) 47 - 52
**[2]** R. Zimmermann, E.R.Rohwer, H.J.Heger, E.W.Schlag, A.Kettrup, G.Gilch, D.Lenoir, U.Boesl: Resonance Ionization Laser Mass Spectrometry: New Possibilities for On-Line Analysis of Waste Incinerator Emissions, Proceedings of the 8. Resonance Ionization Spectroscopy Symposium 1996, *American Institute of Physics (AIP)-Conference Proceedings* 388, AIP-Press New York (1997) 123-126
**[3]** E.K.Ortner, E.R.Rohwer, HRC-J.High Resol. Chromatograph. 19 (1996) 339
**[4]** R.Zimmermann, U.Boesl, H.J.Heger, E.R.Rohwer, E.K.Ortner, E.W.Schlag, A.Kettrup, Hyphenation of Gas Chromatography and Resonance-Enhanced Laser Mass Spectrometry (REMPI-TOFMS): A Multidimensional Analytical Technique, *HRC - J. High Resol. Chromatography 20* (1997) 461-470
**[5]** G. Matz in " Untersuchung der Praxisanforderung an die Analytik beo der Bekämpfung großer Chemieunfälle" Zivilschutz-Forschung, Hrsg.: Bundesamt für Zivilschutz, (ISSN 0343-5164), Neue Folge Band 30
**[6]** H.J.Heger, R.Zimmermann, R.Dorfner, M.Beckmann, H.Griebel, A.Kettrup, U.Boesl, , *Anal. Chem. 71* (1999) *46*
**[7]** T.Gorecki, J.Pawliszyn, Editors: P.Sandra, G.Devos, Procee dings of the 18^{th} Int. Symposium on Capillary Chromatography 1996, Hüthig Verlag, Heidelberg (1996) 762
**[8]** T.Takeuchi, D.Fukuma, J.Matsui, Anal Chem. 71 (1999) 285 - 290
**[9]** S.D.Huang, L.Kolaitis, D.M.Lubman, Applied Spectroscopy 41 (1987) 1371 - 1376
**[10]** Vlasov-Y; Legin-A, FRESENIUS-JOURNAL-OF-ANALYTICAL-CHEMISTRY 361 (1998);: 255-260.
**[11]** Reibel-J; Stier-S; Voigt-A; Rapp-M, ANAL. CHEM. (1998) 70, 5190-5197.
**[12]**
   a) DE 19539589.1
   b) EP 0770870A2
   c) DE 9822672.1
   d) DE19822674.8
**[13]** DE 19754151.5 (1997)
**[14]** F.Hillenkamp, M.Karas, R.C.Bevais, B.T.Chait, Anal Chem 63 (1991) 1196A - 1203A
**[15]** L.Van Vaeck, H.Struyf, W. Van Roy, Fred Adams, Mass Spectrom Reviews. 13 (1994) 198 - 208; ibid.209 - 232
**[16]** L.J.Kovalenko, C.R.Maechling, S.J.Clemett, J.-M.Philippoz, R.N.Zare, C.M.O' D. Alexander, Anal Chem. 64 (1992) 682 - 690
**[17]** A.Hansel, A.Jordan, R.Holzinger, P.Parzeller, W.Vogel, W.Lindinger, Int. J. Mass Spectrom. Ion Processes 149/150 (1995) 609 - 619
**[18]** Georg Schwendt, Analytische Chemie, Georg Thieme Verlag Stuttgart, New York, 1995

## Patentansprüche

1. Verfahren zur Detektion von Spurenstoffen und/oder Umgebungseigenschaften mit folgenden Verfahrensschritten:
a) Auslegen eines in mindestens einer Dimension ausgedehnten Sammelmediums (1) beliebigen Querschnitts nach einem wählbaren Ortsraster.
b) Einholen des Sammelmediums (1) nach einer angemessenen Wechselwirkungszeit mit der Umgebung, z. B. in Luft, Wasser, Boden.
c) Positionsaufgelöste Analyse des Sammelmediums (1) und Korrelation mit dem Ortsraster der Auslegung.

2. Verfahren zur Detektion von Spurenstoffen und/oder Umgebungseigenschaften nach Anspruch 1, **dadurch gekennzeichnet, daß** das Sammelmedium zur positionsaufgelösten Adsorption oder Absorbtion der Spurenstoffe verwendet wird.

3. Verfahren zur Detektion von Spurenstoffen und/oder Umgebungseigenschaften nach Anspruch 1, **dadurch gekennzeichnet, daß** das Sammelmedium (1) als positionsaufgelöster Indikator der lokalen Umgebungseigenschaften verwendet wird.

4. Verfahren zur Detektion von Spurenstoffen und/oder Umgebungseigenschaften nach einem der Ansprüche 1 bis 3, **dadurch gekennzeichnet, daß** die Analyse des Sammelmediums (1) kontinuierlich mittels eines automatisierten Analyseinstruments (5) vorgenommen wird, wobei das Sammelmedium beispielsweise automatisch durch das Analyseinstrument (5) gezogen wird.

5. Verfahren zur Detektion von Spurenstoffen und/oder Umgebungseigenschaften nach einem der Ansprüche 1 bis 4, **dadurch gekennzeichnet, daß** die Analyse des Sammelmediums 1 abschnittsweise erfolgt, wobei die Information über ein bestimmte Länge des Sammelmediums (1) in einem Analysenschritt aufsummiert, beziehungsweise integrativ erfaßt wird.

6. Verfahren zur Detektion von Spurenstoffen und/oder Umgebungseigenschaften nach einem der Ansprüche 1 bis 5, **dadurch gekennzeichnet, daß** das Sammelmedium (1) aus einem sammelaktiven Trägermaterial (3) wie z.B. einer Polymerschnur besteht, das mit einer oder mehreren Schichten gleicher oder verschiedener sammelaktiver Substanzen 2 wie z. B. mit Silikon TENAX oder Aktivkohle beschichtet sein kann.

7. Verfahren zur Detektion von Spurenstoffen und/oder Umgebungseigenschaften nach einem der Ansprüche 1 bis 5, **dadurch gekennzeichnet, daß** das Sammelmedium (1) aus einem nicht sammelaktiven Trägermaterial (3), wie z. B. einem Stahldraht besteht, das mit einer oder mehreren Schichten gleicher oder verschiedener sammelaktiver Substanzen (2) wie z. B. mit Silikon TENAX oder Aktivkohle beschichtet ist.

8. Verfahren zur Detektion von Spurenstoffen und/oder Umgebungseigenschaften nach einem der Ansprüche 1 bis 5, **dadurch gekennzeichnet, daß** das Sammelmedium (1) aus einem sammelaktiven oder nicht sammelaktiven Trägermaterial (3) besteht, das ganz oder teilweise einen Kern aus einer oder mehreren sammelaktiven Substanzen (2) umschließt

9. Verfahren zur Detektion von Spurenstoffen und/oder Umgebungseigenschaften nach einem der Ansprüche 1 bis 8, **dadurch gekennzeichnet , daß** das Sammelmedium (1) aufgewachsene, angelagerte oder eingebettete Mikroorganismen aufweist durch deren Wachstum, Absterben oder Stoffwechselprodukte ein positionsaufgelöster Nachweis chemischer, biologischer oder physikalischer Eigenschaften möglich wird.

10. Verwendung des Verfahrens gemäß einem der Ansprüche 1 bis 9 zum Auffinden von Sprengstoffen oder Minen.

## Claims

1. Process for the detection of trace substances and/or ambient properties, consisting of the following process steps:
a) Laying out, according to a local grid to be selected, of a collection medium (1) that is extended in one dimension at least and has an arbitrary cross section.
b) Recovering of the collection medium (1) after an appropriate time of interaction with the environment, e.g. in air, water, soil.
c) Position-resolved analysis of the collection medium (1) and correlation with the local grid.

2. Process for the detection of trace substances and/or ambient properties according to Claim 1, **characterized by** the collection medium being used for the position-resolved adsorption or absorption of the trace substances.

3. Process for the detection of trace substances and/or ambient properties according to Claim 1, **characterized by** the collection medium (1) being used as a position-resolved indicator of the local ambient properties.

4. Process for the detection of trace substances and/or ambient properties according to one of Claims 1 through 3, **characterized by** the analysis of the collection medium (1) being carried out continuously using an automated analysis instrument (5), with the collection medium, for instance, being drawn automatical through the analysis instrument (5).

5. Process for the detection of trace substances and/or ambient properties according to one of Claims 1 through 4, **characterized by** the analysis of the collection medium (1) taking place in sections, with the information about a certain length of the collection medium (1) being summed up or recorded integratively in an analysis step.

6. Process for the detection of trace substances and/or ambient properties according to one of Claims 1 through 5, **characterized by** the collection medium (1) consisting of a collection-active carrier material (3), e.g. a polymer cord, which may be coated with one or several layers of identical or varying collection-active substances (2), such as TENAX silicone or activated carbon.

7. Process for the detection of trace substances and/or ambient properties according to one of Claims 1 through 5, **characterized by** the collection medium (1) consisting of a non-collection-active carrier material (3), e.g. a steel wire, which is coated with one or several layers of identical or varying collection-active substances (2), such as TENAX silicone or activated carbon.

8. Process for the detection of trace substances and/or ambient properties according to one of Claims 1 through 5, **characterized by** the collection medium (1) consisting of a collection-active or non-collection-active carrier material (3) that completely or partly encloses a core of one or several collection-active substances (2).

9. Process for the detection of trace substances and/or ambient properties according to one of Claims 1 through 8, **characterized by** the collection medium (1) having grown-on, deposited or embedded microorganisms, by the growth, death, or metabolic products of which a position-resolved detection of chemical, biological, or physical properties becomes possible.

10. Use of the process according to one of Claims 1 through 9 for the locating of explosives or mines.

## Revendications

1. Procédé de détection de matières-traces et/ou de caractéristiques du milieu environnant comprenant les phases suivantes:
a) Conception d'un milieu d'échantillonnage (1) étendu du moins dans une dimension, de section quelconque, selon une grille locale à sélectionner.
b) Récupération du milieu d'échantillonnage (1) après un délai d'interaction raisonnable avec l'environnement, p. ex. à l'air, dans l'eau, dans le sol.
c) Analyse résolue en fonction de la position du milieu d'échantillonnage (1) et corrélation avec la grille locale de la conception.

2. Procédé de détection de matières-traces et/ou de caractéristiques du milieu environnant selon la revendication 1, **caractérisé en ce que** le milieu d'échantillonnage est utilisé pour l'adsorption ou l'absorption résolues en fonction de la position des matières traces.

3. Procédé de détection de matières traces et/ou de caractéristiques du milieu environnant selon la revendication 1, **caractérisé en ce que** le milieu d'échantillonnage (1) est utilisé comme indicateur résolu en fonction de la position des caractéristiques locales du milieu environnant.

4. Procédé de détection de matières-traces et/ou de caractéristiques du milieu environnant selon l'une quelconque des revendications 1 à 3, **caractérisé en ce que** l'analyse du milieu d'échantillonnage (1) se fait en continu à l'aide d'un instrument d'analyse automatisé (5), le milieu d'échantillonnage étant prélevé automatiquement par exemple par l'instrument d'analyse (5).

5. Procédé de détection de matières-traces et/ou de caractéristiques du milieu environnant selon l'une quelconque des revendications 1 à 4, **caractérisé en ce que** l'analyse du milieu d'échantillonnage (1) se fait section par section, l'information sur une longueur déterminée du milieu d'échantillonnage étant totalisée ou, respectivement, enregistrée par intégration dans une étape d'analyse.

6. Procédé de détection de matières-traces et/ou de caractéristiques du milieu environnant selon l'une quelconque des revendications 1 à 5, **caractérisé en ce que** le milieu d'échantillonage (1) consiste en un support collecteur actif (3) comme p. ex. un fil polymère qui peut être revêtu d'une ou de plusieurs couches de substances d'échantillonnage actif identiques ou différentes (2) comme p. ex. de silicone TENAX ou de charbon actif.

7. Procédé de détection de matières-traces et/ou de caractéristiques du milieu environnant selon l'une quelconque des revendications 1 à 5, **caractérisé en ce que** le milieu d'échantillonnage (1) est constitué d'un support d'échantillonnage non actif (3), comme p. ex. un fil d'acier, qui est revêtu d'une ou de plusieurs substances d'échantillonnage actif identiques ou différentes (2) comme p. ex. de silicone TENAX ou de charbon actif.

8. Procédé de détection de matières-traces et/ou de caractéristiques du milieu environnant selon l'une quelconque des revendications 1 à 5, **caractérisé en ce que** le milieu d'échantillonnage (1) est constitué d'un support d'échantillonnage actif ou non actif (3) qui renferme totalement ou partiellement un noyau d'une ou de plusieurs substances d'échantillonnage actif (2).

9. Procédé de détection de matières-traces et/ou de caractéristiques du milieu environnant selon l'une quelconque des revendications 1 à 8, **caractérisé en ce que** le milieu d'échantillonnage (1) présente des micro-organismes crus, fixés ou inclus dont la croissance, le dépérissement ou les métabolites permettent la détection, résolue en fonction de la position, des caractéristiques chimiques, biologiques ou physiques.

10. Application du procédé selon l'une quelconque des revendications 1 à 9 pour la détection d'explosifs ou de mines.
